(19) Europäisches Patentamt
European Patent Office
Office européen des brevets

(11) **EP 3 967 156 A1**

(12) **EUROPEAN PATENT APPLICATION**

(43) Date of publication:
**16.03.2022  Bulletin 2022/11**

(21) Application number: **21195125.6**

(22) Date of filing: **06.09.2021**

(51) International Patent Classification (IPC):
**A23L 33/105** (2016.01)       **A61K 36/63** (2006.01)
**C07C 37/70** (2006.01)        **C07C 37/82** (2006.01)
**A23D 7/02** (2006.01)

(52) Cooperative Patent Classification (CPC):
**A23D 7/02; A23L 33/105; A61K 36/63;
C07C 37/70; C07C 37/82**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **07.09.2020  IT 202000021130**

(71) Applicant: **I.T.P. S.r.l. Innovation & Technology
Provider
80121 Napoli (IT)**

(72) Inventors:
• **GARELLA, Isidoro**
  **84013 Cava de' Tirreni (Salerno) (IT)**
• **NIGRO, Federica**
  **80126 Napoli (IT)**

(74) Representative: **Brazzini, Silvia et al
Società Italiana Brevetti S.p.A.
Corso dei Tintori, 25
50122 Firenze (IT)**

(54) **A PROCESS FOR OBTAINING AN EXTRACT COMPRISING OLEOSOMES FROM OLIVE PASTE**

(57)  The present invention relates to a process for the extraction of oleosomes, useful as emulsifiers in food preparations, starting from an intermediate in the production of olive oil, namely olive paste, by means of a simple process, allowing obtainment of oleosomes in high yields with a high polyphenol and flavonoid content, and thus with a high antioxidant power.

**Fig. 1**

EP 3 967 156 A1

**Description**

Field of the invention

The present invention relates in general to the field of processes for the extraction of substances from natural products, and more particularly relates to a process for the extraction of oleosomes, useful as emulsifiers in food, cosmetic and/or pharmaceutical preparations, starting from olive paste.

Background of the Invention

The term *oleosomes,* or *oil bodies,* refers to those lipid-containing structures in plant cells, particularly in seeds but also in other parts of the plants, including leaves and fruits. They are spherical particles, consisting mainly of triacylglycerols surrounded by a layer of phospholipids in which proteins called oleosins and other compounds such as tocopherol are immersed. The phospholipids are arranged with the lipophilic tails inwards and the polar heads outwards, thus creating a true membrane inside which the oleosins help to keep the oleosomes separate from each other, in the form of distinct subcellular structures, like droplets, a true natural oil-in-water emulsion.

The triacylglycerols contained inside the oleosomes consist of a characteristic glycerol portion in which each hydroxyl group is esterified to fatty acid. The structure of triacyl glycerol is always the same, whereas the fatty acids attached thereto change depending on the plant, and in particular their degree of unsaturation, i.e. double bonds, can change, which then affects the chemical and physical characteristics of the oil or of the oleosomes extracted from the plant and also their nutritional value. The higher the proportion of unsaturated fats, which are known to promote the formation of HDL cholesterol and protect against free radicals, compared to saturated fats, which are associated with the risk of cardiovascular disease, the greater the nutritional value of the oil. In this respect, the most appreciable vegetable oil is extra virgin olive oil, due to its high proportion of oleic acid and other unsaturated fatty acids.

The oleosomes represent a real lipid reserve for the plant, used as a source of chemical energy by the plant during the periods of active metabolism, for example during seed germination, when a mechanism of lipid degradation and conversion into sucrose is triggered by oxidative and hydrolytic processes. A degradation of the oleosomes, and thus of the lipids they contain, can also occur when the plants or parts thereof containing oleosomes are subjected to processing that damages the oleosome structures described above. Under normal *in vivo* conditions, oleosomes are stable structures in which all the substances contain therein do not degrade.

Typically, when a vegetable oil is to be obtained from seeds or other parts of a plant that are rich in it, extraction processes are applied in which seeds or fruits are ground and processed to such an extent that the oleosomal structures are destroyed. Thus, the extracted oils will no longer contain oleosomes with their natural emulsion quality, but more or less transformed lipid components, which will also have lost the oxidation stability that characterised them inside the oleosomes.

The possibility of extracting from plant sources the lipid components still inside the intact natural structure represented by the oleosomes has already been the subject of several studies, in order to exploit the natural emulsifying capacity of the oleosomes when mixed with aqueous compounds, even in the absence of other emulsifiers and at room temperature. In the international patent application published under No. WO2007/115899, for example, a method for obtaining oleosomes from oilseeds of various plants, such as sunflower seeds or peanuts, by grinding the seeds, removing the solids and performing extractive washes with different pH buffer solutions has been described. The possibility of loading oleosomes extracted in this way from plant sources with pharmaceutically or cosmetically active ingredients, to formulate vaccines or in industrial food preparations as emulsifying agents has also been described in the literature.

The applications of the oleosomes as emulsifying agents can indeed be very diverse, from the preparation of stable food emulsions to the formulation of water-insoluble active ingredients, such as certain vitamins or nucleotides, in cosmetic or pharmaceutical emulsions. Particularly in the food sector, it is clear that the composition of the lipids inside oleosomes has a bearing on the quality of the final product. In other words, replacing traditional emulsifying additives such as lecithins or carrageenans, which are often blamed for triggering intolerances or gastro-intestinal disorders, with the oleosomes would not only have the advantage of improving the product's tolerability, but could also contribute to the provision of more valuable nutrients, such as unsaturated fatty acids, if already naturally present in oleosomes. Given the ever-increasing attention to the quality of food preparations and the increased sensitivity of consumers to the naturalness of products together with the demand for functional foods, with a positive effect on the body, there is therefore a strong need in the sector for emulsifiers of natural origin to be used as food additives that maintain the organoleptic characteristics of a starting natural product such as the olive, rich in polyphenols and flavonoids as well as a high percentage of unsaturated fatty acids. In the light of what has been explained above, for this purpose a process is required that is capable of extracting intact oleosomes from the starting product, which maintains the organoleptic and nutritional properties, without neglecting the simplicity and industrial scalability of the process.

Summary of the invention

The Applicant has now developed a process for obtaining an oleosome-rich extract starting from olive paste, which solves the problems described above for the known processes by using a special buffer solution, and has the characteristics described in detail below.

In particular, the extraction process of this invention makes it possible to obtain an extract with improved organoleptic properties compared to the products obtainable by known processes, in particular oleosomes having a high content of polyphenols and of flavonoids, which contribute to an improved antioxidant power of the extract that has also shown a high physical, chemical and microbiological stability.

At the same time, the extraction process of this invention is industrially scalable, simpler than the known processes used to date and at the same time capable of providing higher yields of oleosomes.

Subject of this invention is therefore a process for obtaining an extract comprising oleosomes starting from olive paste, whose essential characteristics are defined in the first of the appended claims.

A further subject of this invention is the extract comprising oleosomes obtainable by the aforesaid process, whose essential characteristics are defined by the related independent claim appended thereto.

A further subject of the invention is the use of the aforesaid extract as an additive, in particular as an emulsifying agent, in a food, cosmetic and/or pharmaceutical product, and the food, cosmetic and/or pharmaceutical product comprising them, whose essential characteristics of which are defined in the related independent claims appended thereto.

Other important features of the extraction process, of the extract, and of the use thereof as an additive in food, cosmetic or pharmaceutical products according to the invention are set out in the following detailed description and defined in the dependent claims appended thereto.

Brief description of the figures

Figure 1: microscopic image of an extract obtained by the process of the invention as described in Example 2 reported below; the arrow indicates an oleosomal structure.

Figure 2: microscopic image of the extract of Figure 1 after subjecting the microscope slide to heating at 50°C (a), 100°C (B), 150°C (c) and 200°C (d); the arrow indicates the same oleosomal structure already indicated in Figure 1.

Figure 3: histogram showing the variation with the temperature of the size distributions D10, D50 and D90 of the oleosomes present in the extract of the invention prepared as in Example 2.

Figure 4: viscosity vs. shear rate trends for the three products shown, fresh extract containing oleosomes prepared as in Example 2 (■); extract containing oleosomes after 10 days at 4°C (●); and commercial mayonnaise as a positive control (♦).

Figure 5: Stribeck curve for the same three products tested in Figure 4, obtained as described in the experimental part below.

Detailed description of the invention

In the process of this invention, the starting product used is olive paste, an intermediate in the production of olive oil obtained by pressing olives. To the best of the Applicant's knowledge, this product has never been used before to extract oleosomes; state-of-the-art extraction processes aimed at obtaining oleosomes start from oilseeds.

Within the scope of the present invention, when not otherwise specified, weight percentages of a component are to be understood as referred to the total weight of the composition.

Subject of this invention is a process for obtaining an extract comprising oleosomes starting from olive paste, comprising the following steps:

i) wet grinding of olive paste;
ii) separation and recovery of the oil phase of the ground product;
iii) extraction of said oil phase with an aqueous buffer solution;
iv) separation and recovery of the extract;

said process being characterised in that the starting olive paste is subjected to a freezing step before wet grinding in step i) of the paste, and that the aforesaid aqueous buffer solution has a pH ranging between about 5.0 and about 7.0, preferably a pH of about 6.0, and a density ranging between about 1.1 and about 1.3 g/cm$^3$, preferably a density of about 1.2 g/cm$^3$.

This process, also due to the thermal levels used, allows a rapid thawing without degradative stresses for the antioxidant elements contained in the olive paste, carried out for example by effect of the contact between the thawed paste and the aqueous buffer solution in the grinding step i).

The separation steps ii) and iv) in this process can for example be carried out by centrifugation and simple recovery of the oil phase for further processing.

In the process of this invention, the step i) of wet grinding of the olive paste is preceded by a cryo-concentration pre-treatment, i.e. freezing of the paste, for example by cooling at a temperature comprised between about -20°C and -4°C, preferably at about -4°C. This pre-treatment has a stabilising effect on the starting product, eliminating any problems related to seasonality that might lead to an excessive load on the production process, and also facilitates the separation of the free oil and aqueous phases from the solid components present. As can be seen in the following experimental examples, the yield of the extraction process is significantly increased if the starting material is frozen beforehand, with an improved polyphenol, flavonoid content and antioxidant power. The grinding step obtained with the aforesaid buffer solution and carried out for example at a temperature ranging between about 4°C and about 25°C, preferably at a temperature of about 20°C, also allows thawing the frozen olive paste obtaining an aqueous suspension. The final temperature of this suspension may vary, for example, in a range between about 2°C and about 10°C (in relation to the thermal levels used for mixing) that allows obtaining a suspension at low temperature with consequent limited degradation of the polyphenols and flavonoids initially contained in the olive paste and thus avoiding a decrease in the antioxidant power of the final creamy extract.

In a preferred embodiment of the process of this invention, the same aqueous buffer solution comprising also sucrose in addition to the buffer, used for the subsequent extraction step, may be used as the wet grinding medium. This further simplifies the process, reducing reagents, time and costs of production. The wet grinding techniques that can be used in the context of the present invention are those conventionally used in oil mills, e.g. milling or also techniques using different grinding means, such as pressing, cutting and impact (*i.e.* the use of ceramic or steel balls).

The aqueous buffer solution which can be used for the extraction step in the process of this invention can be chosen, for example, from a citrate or phosphate buffer, preferably it is a citrate buffer, which can be made, for example, from citric acid monohydrate and sodium hydroxide. In one embodiment, the aqueous buffer solution further comprises sucrose.

In order to allow, in the centrifugation step, an easy separation of the extract comprising oleosomes from the aqueous solution, it has proved essential that the density of the aqueous buffer solution, determined for example by the sum of the amounts of the components of the buffer solution and of sucrose if any, be comprised within the interval indicated above, i.e. between about 1.1 and about 1.3 g/cm$^3$, and preferably of about 1.2 g/cm$^3$.

Optimal results in terms of extraction have been observed using the citrate buffer solution added with sucrose. However, any other equivalent buffer solution having a pH and density in the above indicated interval will be within the scope of this invention.

In one aspect, the amount of aqueous buffer solution used in the extraction step may be ranging between about 5.0 and about 10 L/Kg of starting olive paste, and preferably it is of about 7.5 L/Kg.

Compared to the oleosome extraction processes of the prior art, the process of this invention preferably comprises a single step of extraction with a single buffer solution, instead of using several. However, the extraction efficiency and the process yields are higher as a result of this major simplification.

The process of this invention also made it possible to obtain an extract with intact oleosomes derived from olive paste, which maintain its lipid composition and the presence of other substances, which are desirable because they are functional, such as flavonoids and polyphenols. The content of these molecules in the extract was found to be comprised between about 0.70 and about 1.80 mgGAE/g of dry extract for the polyphenols and comprised between about 0.2 and about 0.8 mgQE/g of dry extract for the flavonoids. Furthermore, the antioxidant power shown by the extract obtainable with the present process was found to be comprised between about 12.0 and about 28.0 mgTroloxE/g of dry extract.

The extract comprising oleosomes obtainable with this process, in addition to the stabilising and emulsifying properties characteristic of each type of oleosome, also retains those antioxidant elements present in the olives that can give the final products to which these oleosomes are added a significant nutritional functionality and at the same time a prolonged shelf-life.

The size of the extracted oleosomes, as well as the distribution of this size and the sphericity of the oleosomes, were also assessed after subjecting the extracts to a strong heating, up to 200°C, after which only small variations were found in the three parameters assessed, a sign of great thermal stability of the product obtained by this process.

These, which can generally be identified as the main technical advantages of the present invention, become fundamental positive characteristics for the use of the extracted oleosomes in the field of the food industry, although due to their compositional characteristics and emulsifying capacity they could also be advantageous for the formulation of cosmetic and pharmaceutical active ingredients. In fact, this process uses all food-grade reagents and water as the only solvent, thus being able to aspire to provide an extract that can even be used directly as food for human consumption, without the need for any further purification.

A further advantage of this invention is the ease and speed with which the process can be completed. A much shorter duration than the state of the art of the present process, e.g. about 2 hours, together with the high yields of oleosome extraction, makes the present process suitable for extensive use even on a large scale.

The present invention therefore represents an optimal solution from every point of view, and allows overcoming the drawbacks highlighted above in describing the state of the art.

The following examples are provided for illustrating the present invention but do not constitute a limitation thereof.

Experimental Part

Materials and methods used

The starting product used was olive paste coming from olives of Leccino cultivar olive trees, grown in the area of Perugia, Italy.

The following reagents marketed by Merck were used in the extraction processes:

| Sucrose BioUltra | >99.5% |
| Sodium Chloride BioUltra | >99.5% |
| Citric acid monohydrate | 99.5% |
| Sodium hydroxide | 98% |

The extraction protocol described in the state of the art taken into consideration (International patent application published under No. WO 2007/115899) for the extraction of oleosomes from oilseeds, was repeated here in the comparison Example 1 reported below, by adapting the four operational steps, described in the document, to the olive paste as the starting product. In the first step, wet grinding was carried out using a solution of water and 0.6 M sucrose as grinding medium. The next three steps were carried out using the following aqueous buffer solutions:

Buffer 1: Sucrose (0.2 M) and NaCl (1 M);
Buffer 2: Sucrose (0.1 M) and NaCl (1 M);
Buffer 3: NaCl (0.2 M).

Suspensions were prepared in the following three steps by stirring the material in the step-related buffer solution for 2 minutes, then the suspension was centrifuged at 3226 g for 40 minutes. After each centrifugation, the solid layer was separated and resuspended in the next buffer, then, in the last centrifugation, the yield of the extraction process was assessed as the ratio between the total weight of the extracted product and the initial weight of the olive paste according to the following equation (1):

$$\text{Yield, } \%p = \text{Weight (final extract)/Weight (initial material) (1)}$$

The final product extracted by the state-of-the-art process and by the process of the invention were further both assessed by performing the following analyses, where all the reagents used were purchased from Merck and had laboratory grade purity:

a) Composition (water content, oil content, protein content)

The water content was measured using a thermobalance (Kern DBS 60-3), setting the drying temperature at 60°C. The oil content was determined using instead a continuous Soxhlet extractor filled with 40 g of dried extract and 100 ml of n-hexane. The extraction time was 6 hours. The total protein content of the dried and defatted extract was determined using the Bradford assay method (Bradford M., Anal. Biochem., vol. 72, no. 1-2, pp. 248-254). The protein concentration was determined using a Cary 50, Varian, UV-visible spectrophotometer at 595 nm. BSA (bovine serum albumin) in a concentration interval between 0.1 and 1.4 mg/ml was used as a standard reference.

b) Concentration of polyphenols

For this test, 1 g of extract was diluted in 8 ml of methanol and mixed for 12 hours at 23°C in a test tube sealed with paraffin to prevent evaporation. After centrifugation at 2000 xg for 10 minutes, 1 ml of supernatant was recovered and mixed with 300 $\mu$l of Folin-Ciocalteu reagent and with 1 ml of 99% sodium carbonate (7.5 w/v) and deionised water until a total volume of 10 ml was obtained. The concentration of polyphenols was determined by reading the absorbance curve with a UV-visible spectrophotometer at 765 nm, using gallic acid as a reference in the interval 4.00-25.00 μg/ml (Kim J.S. et al. (2013), J. Funct. Foods, vol. 5, no. 1, pp. 80-86).

c) Concentration of flavonoids

As with the polyphenols, a methanolic extraction was carried out as a first step, then 1 ml of supernatant was mixed with 0.500 μl of 99.9% of aluminium chloride (2% w/v) and deionised water up to a total volume of 10 ml. The solution was then incubated for 30 minutes in the dark and the absorbance at 420 nm was recorded with a UV-visible spectrophotometer. The flavonoid concentration was determined by quantification with quercetin as a standard reference, with a concentration between 4.00 and 20.00 μg/ml (Woisky R.G. et al. (1998), J. Apic. Res.vol. 37, no. 2, pp. 99-105).

d) Antioxidant power

Starting again with a methanolic extraction, the antioxidant power was determined using a FRAP (Ferric Reducing Ability of Plasma) assay: 1 ml of supernatant was mixed with 1 ml of FRAP reagent as described by Benzie I.F.F. et al. (1996) Anal. Biochem., vol. 239, no. 1, pp. 70-76, and distilled water was added up to a total volume of 10 ml. We then incubated the thus prepared solution for 5 minutes and recorded the absorbance at 593 nm with a UV-visible spectrophotometer. The antioxidant power values were measured in Trolox 97% equivalents with concentrations between $1.2 \times 10^{-5}$ and $6 \times 10^{-5}$ mmol/ml and were used as standards.

e) Morphological analysis;

The shape and the size of the oleosomes in the extract were assessed with a Zeiss Axio Imager M1 upright microscope, equipped with a 40X objective, in a reflected light configuration. The extract was placed on a microscope slide and scored with the tip of a pipette, then images of the slide were digitally acquired and analysed using the Fiji-ImageJ software with the particle analysis function and the Nobuyuki Otsu analysis method described in Otsu N. (1979) IEEE Trans. Syst. Man. Cybern., vol. 9, no. 1, pp.62-66. The particle size, the size of the oleosomes and their circular shape were determined. The distribution characteristics of the oleosomes were also assessed according to the values D10, D50 and D90. The median D50 is defined as the diameter below which half the population falls. Analogously, 90% of the population has a diameter below D90 and 10% of the population below D10.

f) Thermal stability of the oleosomes

The thermal stability of the extract containing oleosomes was assessed by measuring the size distribution using the same process as described above under point e) after holding the microscope slide on a hot plate (by Thermo Fisher) prior to the microscopic observation, for 10 minutes after reaching the set temperature. Four tests were carried out at different temperatures equal to 50°C, 100°C, 150°C and 200°C.

g) Emulsion stability

The adopted protocol consisted of measuring the height of the liquid phases, i.e. the oil at the top and the water at the bottom, which separate in the extract, in a 15 ml Falcon tube, after a centrifugation cycle carried out at 2064 g and at a temperature of 25°C. The number of centrifugations performed depended on when a constant stability index was obtained in the assessment, similar to what is done in the food industry to assess the stability of food in emulsion form. The assessment was carried out on the fresh extract and on the extract after heating in a Whirlpool MWF 427 SL microwave oven for 20 seconds up to a temperature of 70°C. The sample was then cooled to room temperature prior to stability assessment.

The stability index % $S_i$ was determined by the following equation (2)

$$S_i = (H_s / H_t) \times 100 \qquad (2)$$

wherein $H_s$ is the sum of the heights of the upper and lower layers measured after each centrifugation and $H_t$ is the total height of the product in the container.

h) Rheological behaviour

The rheological properties of the oleosomes extracted in the experiments described herein were assessed in parallel with a commercial mayonnaise chosen as a positive control, using a rotational rheometer (Anton Paar MCR 102) with a flat-cone geometry (D=50 mm, $\alpha=1°$). Three different tests were carried out:

- flow curve: is a graphical representation of the fluid viscosity when subjected to increasing and decreasing shear rates. The viscosities of the extracts were tested at $t_0$ (fresh extracts) and at $t_1$ (extracts after 10 days at 4°C), in parallel with the viscosity of the commercial mayonnaise. The flow curves were determined using a shear rate interval of 0.1-100 s$^{-1}$ by setting the temperature at 25°C for all tests, then recording the viscosity trend with respect to the shear rate.
- thermal ramp of the flow curve: the behaviour of the oleosome samples at increasing temperatures was investigated with the thermal ramp of the flow curve within a temperature interval of 10-80°C in order to assess the variation in viscosity with heating rate of the samples of 10°C per minute. The lowest value of the shear stress above which the behaviour of a certain material resembles a fluid and below which it behaves like a solid was also determined;
- frequency sweep: differences in the viscoelasticity of the samples were determined by assessing and comparing G' (storage modulus) and G" (loss modulus) at a frequency of 1 Hz, which was chosen for its value representative of the previously assessed viscoelastic linear region.

i) Tribological behaviour (Stribeck curve);

The Stribeck curve was measured using the tribological cell T-PTD 200 mounted on a rotational rheometer (Anton Paar MCR 102); this curve is generally used to assess the friction between two liquid-lubricated surfaces, in this case between the emulsion and a consumer's mouth. The probe of the apparatus was fitted with a glass sphere simulating the tongue and the PDMA tips resembling the palate in the mouth. The resulting curve is divided into three regions, boundary, mixed and hydrodynamic ones, corresponding to different friction coefficients, and different amounts of sample between the tongue and the palate. The tests were carried out at room temperature comparing the behaviour of the fresh extract and of the extract stored for 10 days at 4°C, in parallel with a commercial mayonnaise. The analyses were carried out at a constant pressure between the glass sphere and the tips at a value of 1 N, by varying the flowability rate in the range of 1E$^{-9}$ to 1E$^{-1}$ (m/s).

EXAMPLE 1 - COMPARISON - Extraction of oleosomes according to the process described in WO 2007/115899

The olive paste was frozen at -20°C, then thawed and a 40 g sample was homogenized with a 0.6 M sucrose solution. The protocol described in the above-mentioned state-of-the-art document was then applied exactly, making three successive dilutions of the collected cream in three different liquid buffers, referred to above as Buffer 1, Buffer 2 and Buffer 3, without obtaining any separation of the solid phase from the liquid phase.

Only by adjusting the pH of the suspensions to about 6 and by increasing the concentrations of sucrose and sodium chloride in the buffers as indicated below:

Buffer 1': Sucrose (0.8 M) and NaCl (1 M);
Buffer 2': Sucrose (0.4 M) and NaCl (1 M);
Buffer 3': NaCl (3.2 M),

it was possible to carry out the separation and complete the extraction process, obtaining however a very low yield equal to 21.4%. Moreover, after each centrifugation the liquid phase did not appear clear due to suspended solid particles. In order to recover all the solid product, the container with the suspension had to be frozen each time for about 3 hours, which was unacceptably long and difficult for an industrial process.

EXAMPLE 2 - Extraction of oleosomes according to the process of the invention

The olive paste was frozen at -20°C. 40 g of frozen olive paste was then thawed and homogenized simultaneously with a 0.6 M sucrose solution at a temperature of 20°C, then centrifuged and an aqueous buffer solution was added to the oily suspension recovered after centrifugation, having the following % composition:

- sucrose: 27.4 %
- citric acid monohydrate: 5.2 %
- sodium hydroxide: 2.4 %

The pH of the buffer solution used was 6.0. After suspension and centrifugation, the two phases were again separated, allowing the recovery of 17 g of a cream containing oleosomes and having the desirable characteristics described below in Example 3 (yield = 43%).

The total process time was 2 hours and the amount of reagents used compared to the state-of-the-art process was much reduced, but a much higher yield of oil extract was obtained.

EXAMPLE 3 - Characterisation of the extract of the invention

The extract obtained by the process described above in Example 2 was found to consist mainly of water and lipids, respectively 40% and 26.76% by weight with respect to the total weight of the composition, with 6% by weight of protein component consisting mainly of oleosins and 27.25% by weight of other components consisting mainly of fibres.

Table 1 below shows the polyphenol, flavonoid content and the antioxidant power found for the same extract.

Table 1

| Extract of oleosomes from olive paste Polyphenol and flavonoid content and antioxidant power | |
|---|---|
| Total polyphenols | 1.11 mgGAE/g* |
| Total flavonoids | 0.50 mgQE/g* |
| FRAP | 20.04 mgTroloxE/g* |
| *the amounts are estimated on the weight of the dry extract | |

The values found were surprisingly high, suggesting a great potential of the extract in the food field both in terms of increasing food preservation and in terms of the positive effects of food on consumer health. These values are in fact in line with the corresponding values reported in the literature for extra virgin olive oil with regard to polyphenols, while the antioxidant power is much more stable than that of the oil, suggesting that the emulsions prepared using the extract of this invention could be much more stable.

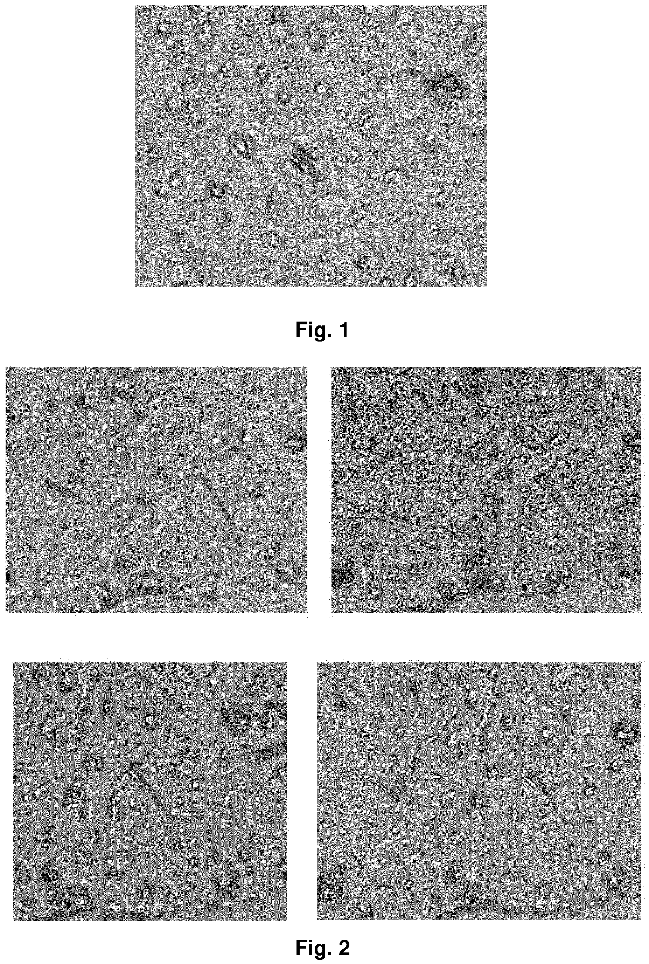

Morphological investigations under the optical microscope revealed the presence of spherical subcellular structures known as oleosomes in the extract. Figure 1 shows an image recorded at room temperature in a light field, where the arrow indicates one of the oleosome structures.

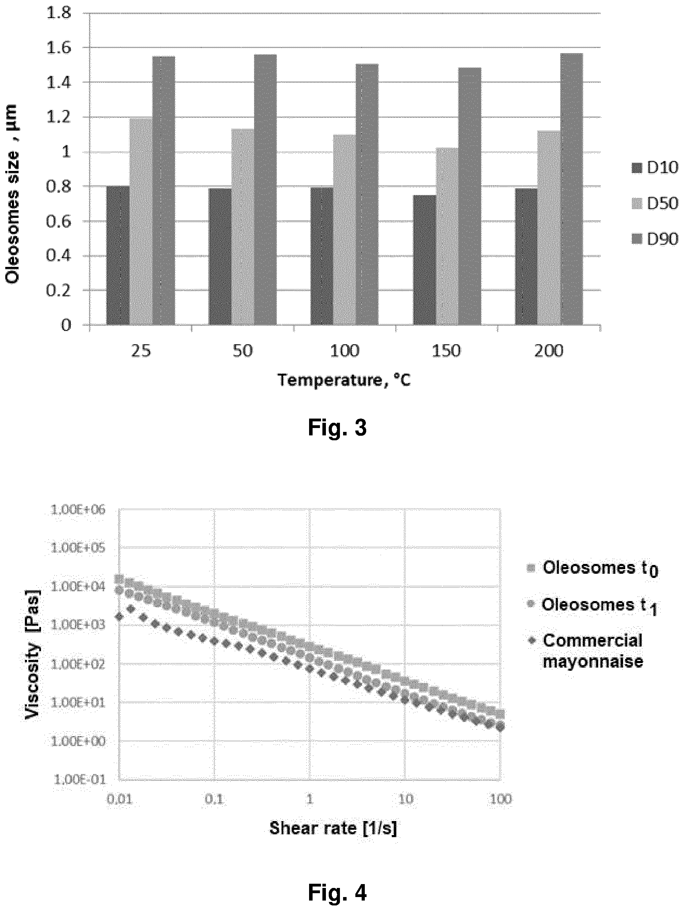

The same microscope slide was again observed after the heat treatments at 50, 100, 150 and 200°C and no significant variations were found in the microscopic structure, as can be seen in Figures 2 a), b), c), and d). This result is also very promising for an industrial use of the extract as it ensures its stability after, for example, heat sterilisation processes. This result was also confirmed by the analysis of the sizes and size distributions of the oleosomes in the extract, which show a remarkable constancy at the different temperatures. Figure 3 shows a histogram of the size parameters D10, D50 and D90 at the temperatures tested, which confirms the stability of this extract to thermal degradation, showing results that are in any case comparable to those obtained at room temperature.

The thermal stability of the product in terms of emulsion was then assessed by observing the degree of separation after centrifugation for the product before and after heating to 70°C. The determined values of the stability index Si are shown in Table 2 below:

Table 2

| No. of centrifugation step | Time (minutes) | Degree of separation % of the fresh extract | Degree of separation % of the heated extract |
|---|---|---|---|
| 1 | 5 | 1.1 | 0 |
| 2 | 10 | 1.1 | 0 |
| 3 | 15 | 2.2 | 3.8 |
| 4 | 20 | 3.4 | 4.6 |
| 5 | 25 | 4.5 | 5.8 |
| 6 | 30 | 4.5 | 6.9 |

As can be seen from these results, the values of the degree of separation do not vary much even after 6 centrifugation passages, both for the fresh extract and for the one treated with heating.

Rheological measurements were also carried out on the extract as described above. Figure 4 shows, for example, the trend found for the viscosity with respect to the shear rate for the fresh extract and the time t1 defined above, in a comparison with a commercial mayonnaise chosen as a benchmark. There is a close similarity with the commercial product again for the extract after 10 days storage at 4°C, and a higher viscosity.

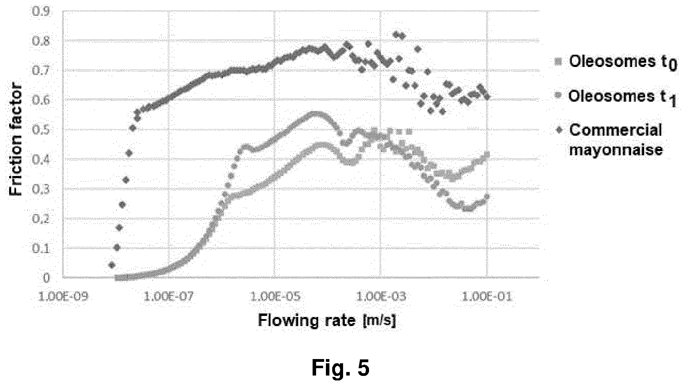

Finally, the tribological tests simulating the transformation of the product into a bolus, also carried out in parallel with a commercial mayonnaise, highlighted a lower friction factor than the latter, thus suggesting that the oleosomes extracted by this process from the olive paste are easier to swallow than a mayonnaise. Figure 5 shows the Stribeck curves recorded for the commercial mayonnaise chosen as a positive control and for the two extracts, fresh and after 10 days storage.

EXAMPLE 4 - Extraction of oleosomes according to the process of the invention

The olive paste was frozen at -20°C, then a 40 g sample was thawed and homogenized directly in a solution at a temperature of 20°C with the following % composition:

- sucrose: 27.4 %
- citric acid monohydrate: 5.2 %
- sodium hydroxide: 2.4 %

After centrifugation, a buffered aqueous solution with the same percentage composition as above was added to the recovered oily suspension.

The pH of the buffer solution used was 6.0. After suspension and centrifugation, the two phases were again separated, allowing the recovery of 17 g of a cream containing oleosomes and having the desirable characteristics described below in Example 3 (yield = 42.9%).

Also in this case, the total process time was 2 hours and the amount of reagents used compared to the state-of-the-art process was much reduced, but a much higher yield of oil extract was obtained, substantially the same obtained by following the process of EXAMPLE 2 reported above.

The polyphenol and flavonoid content and the antioxidant power returned values comparable to those obtained with the above reported process in EXAMPLE 2.

The concentrations of polyphenols, flavonoids and the antioxidant power measured in this case are similar to those obtained with the process in EXAMPLE 2 and reported in EXAMPLE 3 above, and are: 1.09 mgGAE/g, 0.48 mgQE/g and 19.82 mgTrolox/g.

EXAMPLE 5 - COMPARISON - Extraction of oleosomes without freezing pre-treatment

An amount of 40 g of olive paste milled for 1 day and refrigerated at 4°C was homogenized, as in the case of EXAMPLE 4 reported above, directly in a solution at a temperature of 20°C having the following % composition:

- sucrose: 27.4%;
- citric acid monohydrate: 5.2%;
- sodium hydroxide: 2.4%;

then centrifuged and the same buffered aqueous solution was added to the oil phase recovered after centrifugation and characterised by the same composition reported above in EXAMPLE 4.

The pH of the buffer solution used was 6.0. After suspension and centrifugation, the two phases were again separated, allowing the recovery of 13.5 g of a cream containing oleosomes with a yield equal to = 33.8% lower than that obtainable with the initial freezing of the olive paste, as shown in EXAMPLE 4 above. The chemical and physical characteristics of the extracted oleosome cream are substantially unchanged, except that the polyphenol and flavonoid content and the antioxidant power have been reduced to the following values respectively: 0.82 mgGAE/g, 0.39 mgQE/g and 12.72 mgTrolox/g.

Both the total process time and the amount of reagents were the same as those used in EXAMPLE 4 with significantly reduced amounts compared to the state of the art with extraction yields of the oleosome cream aligned with the state of the art.

The present invention has been described herein with reference to a preferred embodiment. It is to be understood that there may be other embodiments that relate to the same inventive nucleus, all falling within the scope of protection of the claims provided below.

**Claims**

1. A process for obtaining an extract comprising oleosomes starting from olive paste, comprising the following steps:

    i) wet grinding of olive paste;
    ii) separation and recovery of the oil phase of the ground product;
    iii) extraction of said oil phase with an aqueous buffer solution;
    iv) separation and recovery of the extract;

    said process being **characterised in that** said olive paste is subjected to a freezing step before said wet grinding with subsequent thawing, and **in that** said aqueous buffer solution has a pH ranging between 5.0 and 7.0, and a density ranging between 1.1 and 1.3 g/cm$^3$.

2. The process of claim 1, wherein said aqueous buffer solution has pH 6.0.

3. The process of claim 1 or 2, wherein said aqueous buffer solution is a solution in water of citric acid and sodium hydroxide.

4. The process of any one of the preceding claims, wherein said aqueous buffer solution further comprises sucrose.

5. The process of any one of the preceding claims, wherein said aqueous buffer solution has a density equal to 1.2 g/cm$^3$.

6. The process of any one of the preceding claims, comprising a single step of extraction by washing with said aqueous buffer solution.

7. The process of any one of the preceding claims, wherein said wet grinding step is carried out by homogenization of said olive paste with the same aqueous buffer solution comprising sucrose of the extraction step, as grinding medium.

8. The process of any one of the preceding claims, wherein said thawing of the frozen olive paste is carried out by effect of the contact with said aqueous buffer solution during said grinding step.

9. Extract from olive paste comprising oleosomes obtainable by the process as defined in claims 1-8, having a preservative and stabilising effect due to a polyphenol content comprised between 0.70 and 1.80 mgGAE/g of dry extract, an amount of flavonoids ranging between 0.20 and 0.80 mgQE/g of dry extract, and an antioxidant power content ranging between 12.0 and 28.0 mgTroloxE/g of dry extract.

10. Use of the extract of claim 9, as emulsifying agent in food preparations, in cosmetic and/or pharmaceutical formulations.

Fig. 1

Fig. 2

**Fig. 3**

**Fig. 4**

**Fig. 5**

Europäisches Patentamt

European Patent Office

Office européen des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 986 309 B1 (SEMBIOSYS GENETICS INC [CA]) 20 February 2008 (2008-02-20) * paragraph [0001] – paragraph [0128] * ----- | 1-10 | INV. A23L33/105 A61K36/63 C07C37/70 |
| A | US 2019/105248 A1 (YANG SOO IN [CA] ET AL) 11 April 2019 (2019-04-11) * paragraph [0002] – paragraph [0197]; examples * ----- | 1-10 | C07C37/82 A23D7/02 |
| A | HOU JUNCAI ET AL: "Effect of NaCl on oxidative stability and protein properties of oil bodies from different oil crops", LWT- FOOD SCIENCE AND TECHNOLOGY, ACADEMIC PRESS, UNITED KINGDOM, vol. 113, 14 June 2019 (2019-06-14), XP085736680, ISSN: 0023-6438, DOI: 10.1016/J.LWT.2019.108263 [retrieved on 2019-06-14] * Abstract; p.2 3.2 * ----- | 1-10 | |
| A | BERGFELD R. ET AL: "Formation of oleosomes (storage lipid bodies) during embryogenesis and their breakdown during seedling development in cotyledons of Sinapis alba L.", PLANTA, vol. 143, no. 3, 1 January 1978 (1978-01-01), pages 297-307, XP055884703, Berlin/Heidelberg ISSN: 0032-0935, DOI: 10.1007/BF00392002 * page 298, column 2, last paragraph; figure 7 * * page 306, column 2, paragraph 3 * ----- | 1-10 | TECHNICAL FIELDS SEARCHED (IPC) A23D C11C A61K C07C A23L |

-/--

The present search report has been drawn up for all claims

| Place of search | Date of completion of the search | Examiner |
|---|---|---|
| Munich | 28 January 2022 | Alevisopoulos, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 107 129 859 A (UNIV CHONGQING EDUCATION) 5 September 2017 (2017-09-05) * paragraph [0029] - paragraph [0030] * ----- | 1-10 | |
| A | WO 2008/122320 A1 (MEDITERRANEA IDENTITAT S L [ES]; MARQUEZ GOMEZ AMADOR [ES] ET AL.) 16 October 2008 (2008-10-16) * page 3, line 22 - line 29 * * page 7, line 8 - line 34 * ----- | 1-10 | |
| A | ROSS J H E ET AL: "Differential presence of oleosins in oleogenic seed and mesocarp tissues in olive (Olea europaea) and avocado (Persea americana)", PLANT SCIENCE, ELSEVIER IRELAND LTD, IE, vol. 93, no. 1-2, 8 June 1993 (1993-06-08) , pages 203-210, XP025475926, ISSN: 0168-9452, DOI: 10.1016/0168-9452(93)90050-A [retrieved on 1993-01-01] * Abstract; p.204: 2.1, 2.3 * ----- | 1-10 | |
| A | MURPHY D J ET AL: "Purification and immunogold localisation of the major oil-body membrane protein of oilseed rape", PLANT SCIENCE, ELSEVIER IRELAND LTD, IE, vol. 60, no. 1, 15 September 1988 (1988-09-15), pages 47-54, XP025400975, ISSN: 0168-9452, DOI: 10.1016/0168-9452(89)90042-3 [retrieved on 1989-01-01] * p.48, "Isolation of oil-bodies" * ----- | 1-10 | |

TECHNICAL FIELDS SEARCHED (IPC)

The present search report has been drawn up for all claims

| Place of search | Date of completion of the search | Examiner |
|---|---|---|
| Munich | 28 January 2022 | Alevisopoulos, S |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 21 19 5125

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2022

| Patent document cited in search report | | Publication date | Patent family member(s) | | Publication date |
|---|---|---|---|---|---|
| EP 0986309 | B1 | 20-02-2008 | AR | 012877 A1 | 22-11-2000 |
| | | | AT | 386439 T | 15-03-2008 |
| | | | AU | 737896 B2 | 06-09-2001 |
| | | | BR | 9809691 A | 03-10-2000 |
| | | | CA | 2291274 A1 | 03-12-1998 |
| | | | CN | 1258198 A | 28-06-2000 |
| | | | CY | 1107375 T1 | 19-12-2012 |
| | | | DE | 69839148 T2 | 12-02-2009 |
| | | | DK | 0986309 T3 | 09-06-2008 |
| | | | EP | 0986309 A1 | 22-03-2000 |
| | | | ES | 2301197 T3 | 16-06-2008 |
| | | | HK | 1027487 A1 | 19-01-2001 |
| | | | IL | 132908 A | 25-07-2002 |
| | | | JP | 3869861 B2 | 17-01-2007 |
| | | | JP | 2002503268 A | 29-01-2002 |
| | | | KR | 20010013047 A | 26-02-2001 |
| | | | KR | 20040101211 A | 02-12-2004 |
| | | | NO | 316731 B1 | 19-04-2004 |
| | | | NZ | 501404 A | 28-09-2001 |
| | | | PT | 986309 E | 03-04-2008 |
| | | | TW | 505508 B | 11-10-2002 |
| | | | US | 6146645 A | 14-11-2000 |
| | | | US | 6210742 B1 | 03-04-2001 |
| | | | WO | 9853698 A1 | 03-12-1998 |
| US 2019105248 | A1 | 11-04-2019 | CA | 3020765 A1 | 19-10-2017 |
| | | | EP | 3442489 A1 | 20-02-2019 |
| | | | US | 2019105248 A1 | 11-04-2019 |
| | | | WO | 2017177334 A1 | 19-10-2017 |
| CN 107129859 | A | 05-09-2017 | NONE | | |
| WO 2008122320 | A1 | 16-10-2008 | AU | 2007350730 A1 | 16-10-2008 |
| | | | BR | PI0721476 A2 | 14-10-2014 |
| | | | CA | 2682866 A1 | 16-10-2008 |
| | | | CN | 101707943 A | 12-05-2010 |
| | | | EP | 1978078 A1 | 08-10-2008 |
| | | | EP | 2150604 A1 | 10-02-2010 |
| | | | JP | 2010532156 A | 07-10-2010 |
| | | | RU | 2009140735 A | 10-05-2011 |
| | | | US | 2010178412 A1 | 15-07-2010 |
| | | | WO | 2008122320 A1 | 16-10-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007115899 A

**Non-patent literature cited in the description**

- **BRADFORD M.** *Anal. Biochem.,* vol. 72 (1-2), 248-254
- **KIM J.S. et al.** *J. Funct. Foods,* 2013, vol. 5 (1), 80-86
- **WOISKY R.G. et al.** *J. Apic. Res.,* 1998, vol. 37 (2), 99-105
- **BENZIE I.F.F. et al.** *Anal. Biochem.,* 1996, vol. 239 (1), 70-76
- **OTSU N.** *IEEE Trans. Syst. Man. Cybern.,* 1979, vol. 9 (1), 62-66